# EUROPEAN PATENT SPECIFICATION

(11) **EP 1 326 664 B1**
(45) Date of publication and mention of the grant of the patent: **09.08.2006**
(21) Application number: 01972950.8
(22) Date of filing: 26.09.2001
(51) Int. Cl.: A61M 5/315, A61M 5/24

(54) **PEN DEVICE FOR ADMINISTRATION OF PARATHYROID HORMONE**
STIFTVORRICHTUNG ZUR VERABREICHUNG DES PARATHYROID-HORMONS
DISPOSITIF DU TYPE STYLO POUR L'ADMINISTRATION D'HORMONE PARATHYROIDE

(30) Priority: 09.10.2000 US 239172 P
(43) Date of publication of application: 16.07.2003
(73) Proprietor: ELI LILLY AND COMPANY, Indianapolis, Indiana 46285 (US)
(72) Inventor: ROE, Michael, Joseph, Zionsville, IN 46077 (US)
(74) Representative: Kent, Lindsey Ruth
(86) International application number: PCT/US2001/027732
(87) International publication number: WO 2002/030495

(56) References cited:
- EP-A- 0 554 996
- WO-A-98/55168
- DE-A- 4 112 259
- DE-A- 19 838 760
- US-A- 6 001 089

## Description

### BACKGROUND OF THE INVENTION

This invention relates to devices and methods for the administration of parathyroid hormone (PTH). PTH is a secreted, 84 amino acid product of the mammalian parathyroid gland that controls serum calcium levels through its action on various tissues, including bone. The N-terminal 34 amino acids of bovine and human PTH (PTH(1-34)) is deemed biologically equivalent to the full length hormone. Other amino terminal fragments of PTH (including 1-31 and 1-38 for example), or PTHrP (PTH-related peptide/protein) or analogues of either or both, that activate the PTH/PTHrP receptor (PTH1 receptor) have shown similar biologic effects on bone mass, although the magnitude of such effects may vary.

PTH and PTH fragments can be administered by subcutaneous injection using a conventional syringe-and-vial system, wherein the liquid PTH solution is manually drawn up from a vial using a syringe. However, it is often difficult to draw up the correct amount of liquid using conventional syringes.

Because most osteoporotics are of advanced age, a simple step-by-step procedure for dosing PTH is needed.

### SUMMARY OF THE INVENTION

A need exists to provide the osteoporotic patient with a convenient and simple administration device that allows for the daily dosing of a single, predetermined fixed dose of PTH. The present invention accomplishes this by providing a pen-type injection device designed so that only a single predetermined dose is easily set. Moreover, the present invention involves a pen device containing a reservoir of PTH by which a simple step-by-step method for administering the PTH that virtually eliminates the concern of over or under dosing may be performed. By practicing the invention, PTH is easily administered and results in increased toughness or stiffness at a site of a potential trauma, such as a hip or spine of a person with osteoporosis, or at another site having abnormally low bone mass or poor bone structure.

The present invention provides a pen-type injection or delivery device for administering a single predetermined dose of a solution containing parathyroid hormone. The inventive device is in the general shape of a writing pen having a reservoir containing a solution of PTH. The elongate, compact shape of the pen delivery device facilitates the use of simple injection techniques and allows the patient to easily carry the device on his/her person. The pen device is designed so that only a single predetermined dose is easily set by the patient using a rotatable dose knob attached to the proximal end of the pen. This dose knob can be dialed in both a forward and reverse direction without causing a dose to be delivered. Although the dose knob freely rotates, only one priming position and one dose position is easily set. By having only one dose setting, this greatly simplifies the procedure for administering an injection of PTH. Moreover, having only a single dose setting greatly reduces the possibility of a patient under dosing or overdosing himself or herself.

In one embodiment of the invention, the pen device is disposable and has a reservoir of PTH permanently fixed within the injector body. When the reservoir of PTH is exhausted the entire device is simply thrown away. This reservoir may consist of a glass cartridge permanently sealed within the injector body. In another embodiment, the pen is reusable and the reservoir comprises a replaceable cartridge. Once the PTH in the cartridge is expelled through multiple injections, the empty cartridge is removed from the pen and a new cartridge filled with fresh PTH solution is placed in the injector body. In all cases, the pen is designed so that the pen needle is easily removed by the user facilitating single use of the needle.

### BRIEF DESCRIPTION OF THE DRAWINGS

FIG. 1 is a perspective view of one embodiment of a pen delivery device in accordance with the present invention;
FIG. 2 is an exploded view of the device of FIG. 1;
FIG. 3 is an enlarged perspective view of a portion of the pen device of FIG. 1, particularly showing the dose knob and housing components;
FIG. 4 is an enlarged longitudinal sectional view of a portion of the medication dispensing device of FIG. 1, particularly showing the button assembly disposed within dose knob;
FIG. 5 is an enlarged perspective view, in partial section, of the medication dispensing device of FIG. 1, particularly showing the button assembly disposed in the dose knob;
FIG. 6 is an enlarged cross sectional view of the medication dispensing device of FIG. 1, particularly showing the insufficient remaining dose stop on the nut approaching the corresponding stop on the leadscrew;
FIG. 7 is a view of FIG. 6, except that the insufficient remaining dose stop on the nut is in engagement with the stop on the leadscrew;
FIG. 8 is a perspective view, in partial section, of a housing part in engagement with the dose knob, particularly showing the unit click finger in the zero position;
FIG. 9 is a view of FIG. 8, except that the unit click finger is behind the end-of-dose flange;
FIG. 10 is a view of FIG. 8, except that the unit click finger is shown in the dial splines during dosing;
FIG. 11 is an enlarged sectional view of a portion of the medication dispensing device of FIG. 1, particularly showing the relationship among the button assembly, dose knob, and housing while the device is at the end of dose position;
FIG. 12 is a longitudinal sectional view of the medication dispensing device of FIG. 1, particularly showing the dose knob after it has been rotated to the zero position;
FIG. 13 is a view of FIG. 12 except that the dose knob has been retracted so that the splines of the nut are engaged by the splines of the dose knob;
FIG. 14 is a view of FIG. 13, except that a desired dosage has been dialed up;
FIG. 15 is a view similar to FIG. 11, showing the dose knob rotated 180°, and further showing the button initially depressed before dial movement takes place;
FIG. 16 is a view of FIG. 15, showing the dial having moved forward a small distance;
FIG. 17 is a view of FIG. 15, showing the dial having moved forward half of a thread pitch; and
FIG. 18 is a view of FIG. 14, except that the pen is shown in its end-of-dose position.

### DETAILED DESCRIPTION OF PREFERRED EMBODIMENTS

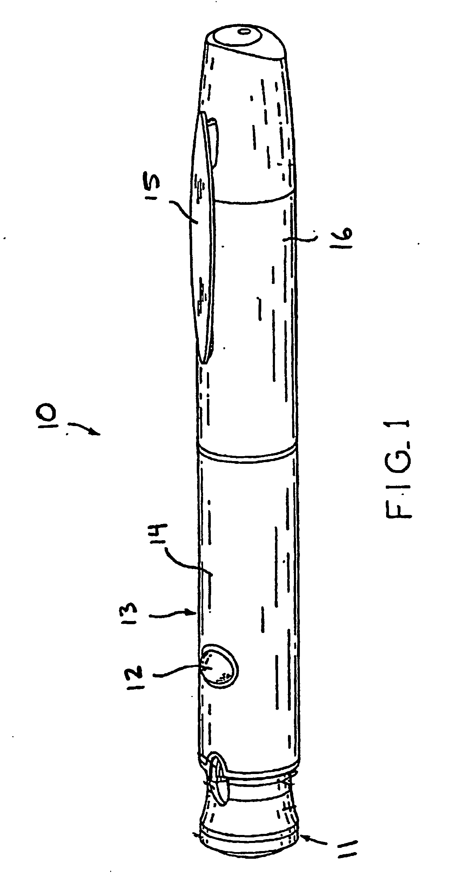

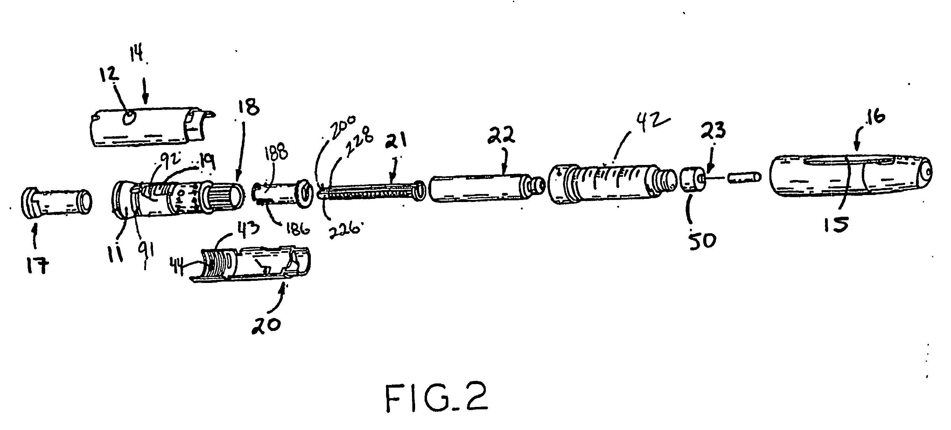

The pen device of the present invention is generally illustrated in FIG. 1. The pen device 10 is in the shape of a standard writing pen and has a clip 15 attached to cap 16, which allows the pen to be securely fastened to a pocket or other carrying means. Cap 16 protects the pen needle 23, as illustrated in FIG.2, and provides access to the reservoir or cartridge 22. To achieve the single dosing feature of the invention, the injector body of the pen device is designed with a hard stop. Although it is not critical where in the pen device the hard stop is located, it must, however, be positioned such that the dose knob 11 cannot be rotated outwardly to a position where the pen will deliver a dose greater than a predetermined single maximum dosage. In the situation where PTH is being prescribed to treat osteoporosis, a set single daily dose of 80 *µ*L has been determined to be effective. Preferably, the hard stop is positioned in either housing 20 or 14 of the injector body and more specifically it is located within threads 43, which are located on the interior portion of both housing 20 and 14. Placing the hard stop within threads 43 ensures that dose knob 11 is prevented from advancing outwardly a distance more than that required to deliver a predetermined single dose. As described in more detail below, the dose knob, after dialing it outwardly to a preset position, is pushed inwardly causing drive stem 21 to advance forward in the distal direction causing the solution of PTH to be expelled through the pen needle.

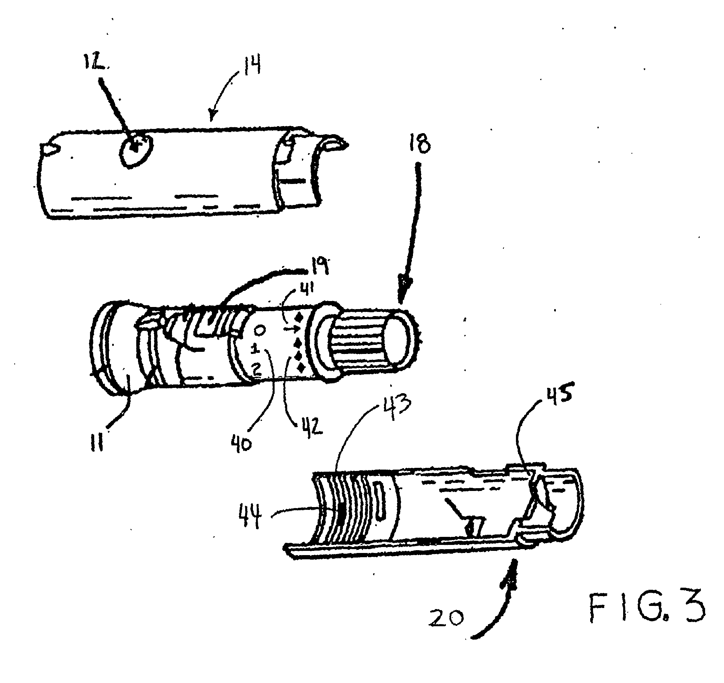

To further ensure that only a single accurate dose is delivered to the patient, the dose knob 11 of the pen device is calibrated and visibly marked with a single priming position indicator and a single predetermined injectable dose position indicator. This calibration and marking of the dose knob is a significant departure from existing pen delivery devices typically used for insulin or human growth hormone delivery. Those existing devices are characterized in that they can deliver a great number of different dosages depending on where the patient or health care provider dials the dose knob. Moreover, the dose knobs of those existing devices are marked and calibrated in International Units, typically having up to 80 different possible dose settings. One of much devices is disclosed in document US 6,001,089. In contrast, the administration of PTH typically requires only a single, predetermined, non-varying dose on a daily basis. Accordingly, device 10 is not marked with International Units, but instead with three discrete step indicators. Preferably these steps indicators are printed on the dose knob as "0," "1," and "2." These step indicators are illustrated in FIG. 3 by numeral 40. Step indicators "1" and "2" correspond to a predetermined priming dose and an injectable dose, respectively.

In addition to the pen device, there is also described a method or procedure for performing an injection of PTH. That method involves rotating the dose knob 11 until an arrow ("→") 41, or other identifying symbol, printed on the dose knob 11 is visible to the patient through lens 12 in housing 14. Once the arrow is in position, dose knob 11 can be pulled outwardly (towards the proximal end of the pen) until the first step or starting position is reached, designated by the "0" visible through lens 12. The "0" step indicator signals the user that dial knob 11 of the pen device is correctly positioned to continue the procedure for delivering a single predetermined dose of PTH. Step designations "0," "1," and "2" are printed on dose knob 11 and viewed by the patient through lens 12. After the "0" starting position is reached, the patient further rotates the dose knob 11 until the priming position is reached. A printed "1" appears in the lens designating that the second position has been reached. The user then pushes on disengaging device 17 to cause the dose knob and drive stem to move inwardly (towards the distal end of the pen) expelling a small amount of PTH from the needle. Typically, the priming dose is predetermined to be approximately 10-20 µL. The priming dose is an important step in the method because it allows the patient to verify that the pen is working properly and that the pen needle is positioned correctly and not clogged or obstructed.

Once priming is completed, the next step in the method involves repeating the first two steps of rotating the dial knob 11 until the arrow ("→") 41 comes into view in lens 12, pulling the knob outward until the "0" is visible and then rotating the dose knob past the "1" indicator position to the third and last position, designated by the number "2." Although it is preferred to use Arabic numerals, any logical sequence of symbols, such as Roman numerals or letters will provide the necessary visual feedback to the end user signaling the three positions. As with the priming position, the injectable dose position is predetermined and corresponds to a dose which is customary for the treatment of a specific disorder using PTH. In the case of osteoporosis, it is common to prescribe a daily dose of 80 µL. As mentioned, to ensure that the injectable dose does not exceed the predetermined quantity of PTH, the pen device is designed with a hard stop that prevents the dose knob from being dialed outwardly past the "2" position. The exact location or design of the hard stop is not critical to the invention, as long as the dose knob is prevented from rotating past the predetermined injectable dose position. A preferred hard stop is one that is placed within threads 43 of either housing 20 or 14 as illustrated in FIGS. 2 and 3 by numeral 44. The hard stop can be made of any material that will stand up to repetitive contact with finger 19 of dose knob 11. In operation of the pen device, finger 19 engages threads 43 located inside housings 14 and 20 allowing the dose knob 11 to move inwardly and outwardly as it is rotated from the "0" position. As the dose knob is rotated outwardly, finger 19 eventually will come into contact with hard stop 44, thus preventing any further rotation. Preferably, hard stop 44 is made of the same plastic material that is used to construct the housings. The exact positioning of the hard stop within the threads is selected so that the dose knob, using the dose knob, cannot be rotated outwardly past injectable dose position "2."

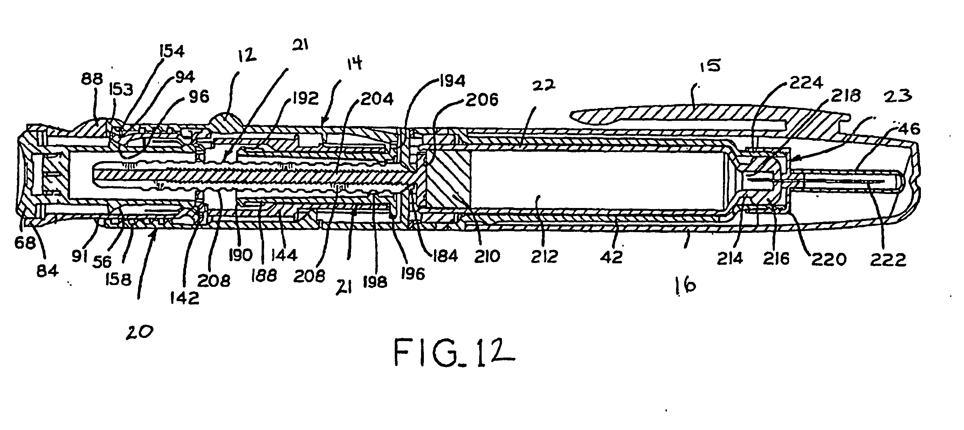

Once position "2" is reached, the patient is ready to insert pen needle 23 into the injection site. The pen needle of device 10 can be any type of pen needle known to the art, typically comprising a double-ended needle, fixed in hub 50 that can be easily screwed on and off the distal end of the pen assembly. After insertion of the pen needle, the patient depresses disengaging device 17. When disengaging device 17 is pushed inwardly, dose knob 11 and drive stem 21 also move inwardly in a linear direction towards the distal end of the pen device. As drive stem 21 moves inwardly it pushes on a piston 210 (see Figs. 12 -14) within the reservoir containing the PTH solution, shown in FIG. 2 as cartridge 22. As the piston is pushed by drive stem 21 towards the distal end, it causes the solution of PTH to be expelled through pen needle 23. To signal the end-of-dose, the invention provides both visual and tactile indicators. The visual indicator is viewed by the patient through lens 12, as a distinct end-of-dose symbol, such as a diamond shape ("◇") 42, which is printed on the dose knob. If the patient does not see the symbol he knows to continue pushing on the disengaging device 17. The tactile indicator comprises a second hard stop, which is the point of contact between the dose knob 11 and the bulkheads 45 on housings 14 and 20. The contact between the dose knob and the bulkheads on the housings prevents further travel of the drive stem and provides a tactile feel to the patient indicating the dose is complete.

The invention also contains a mechanism that will prevent the dose knob from being rotated to injectable dose position "2" if there is an insufficient solution of PTH remaining within the reservoir of the pen device. This feature is accomplished by predetermining the distance the drive stem must travel to deliver the contents of the reservoir. In the case where a glass cartridge is used as a reservoir, typically this would be about 3 mL. Of course, this volume is dependant on the cartridge's dimensional tolerances and can vary accordingly. The drive stem is designed with only a sufficient number of threads to allow the dose knob to dial and deliver a discreet number of doses. Since the dose knob moves towards the distal end of the threads on the drive stem 21 as the dose knob 11 is rotated outwardly to set a dose, and the drive stem is prevented from moving backward after each injection by anti-backup features, which are described more fully below, the dose knob gets closer to the end of the drive stem's threads with each injection. When the amount of starting PTH solution has been expelled, the drive stem will be at the end of its threads. To further illustrate this feature, consider the example where the reservoir has only 40 µL of PTH solution remaining after an injection, and the user attempts to perform another injection of 80 µL. Following the administration procedure, the following would occur. The user would be able to correctly perform the steps through the priming position "1," however, they would not be able to rotate the dose knob to the third position "2," this is because only 20 µL remain in the reservoir (40 µL - 20µL (priming dose)). Since the threads on the drive stem correspond to the volume of PTH solution remaining in the reservoir, no further travel of the dose knob about the threads of the drive stem is possible, hence no further rotation of the dose knob is possible. As such, the user is unable to rotate the dose knob to a position where the "2" is visible, thus signaling the user that the pen device is not properly set for administration of the correct predetermined dose. At this point, the user would either discard the pen, if it was disposable, or would replace the cartridge with a full one.

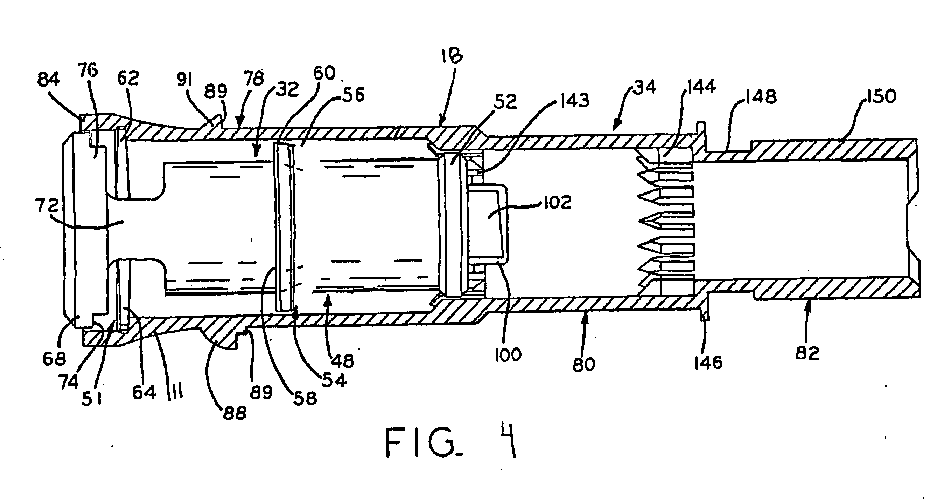

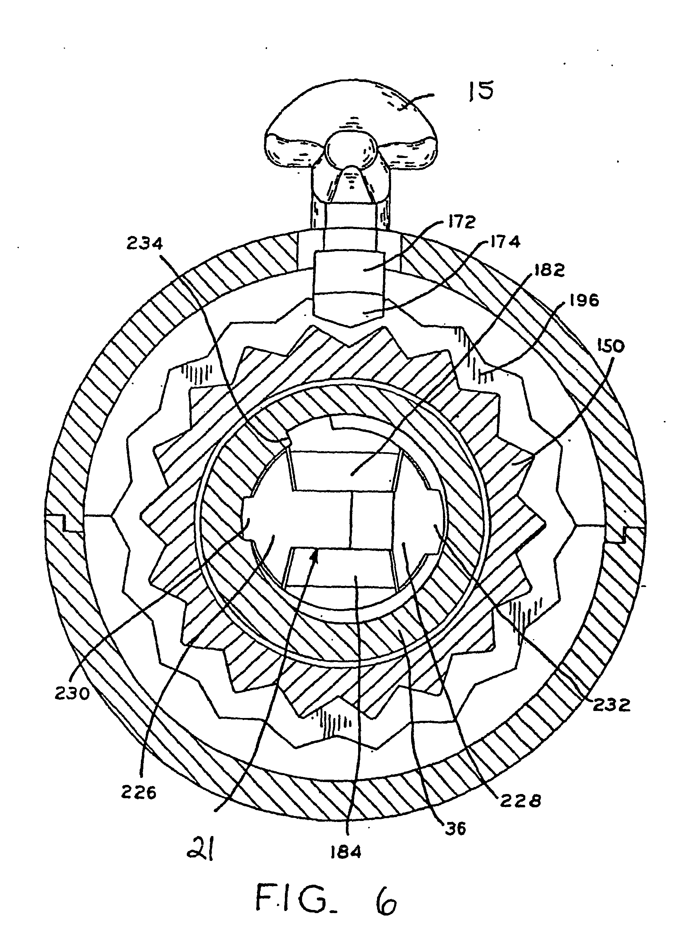

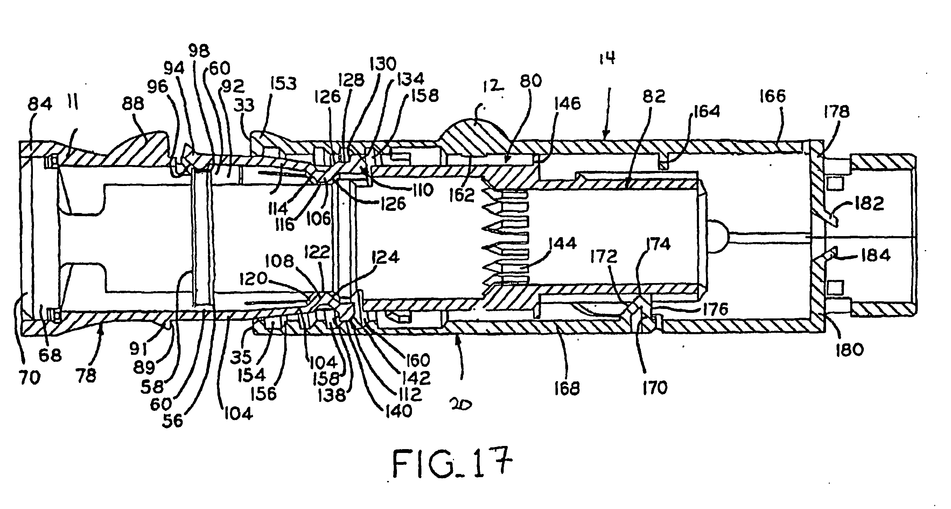

FIGS. 4-18 provide a more detailed and specific mechanical configuration of the pen device and for purposes of this application, the term "proximal" shall designate a relative axial position toward the knob end of the delivery mechanism, and the term "distal" shall designate a relative axial position toward the delivery needle end of the delivery mechanism. All of the components of medication device 10, except cartridge 22 and needle 23 may be made of a plastic material that is suitable for recycling. Suitable plastics include high flow polycarbonates resins, which can be processed by conventional injection molding and extrusion. In one embodiment, the housing parts 14, 20 and distal body 42 are made from an optically clear polycarbonate material, and the remaining plastic components are made from ABS resins. These plastics are recyclable, thereby making disposal of the device environmentally desirable.

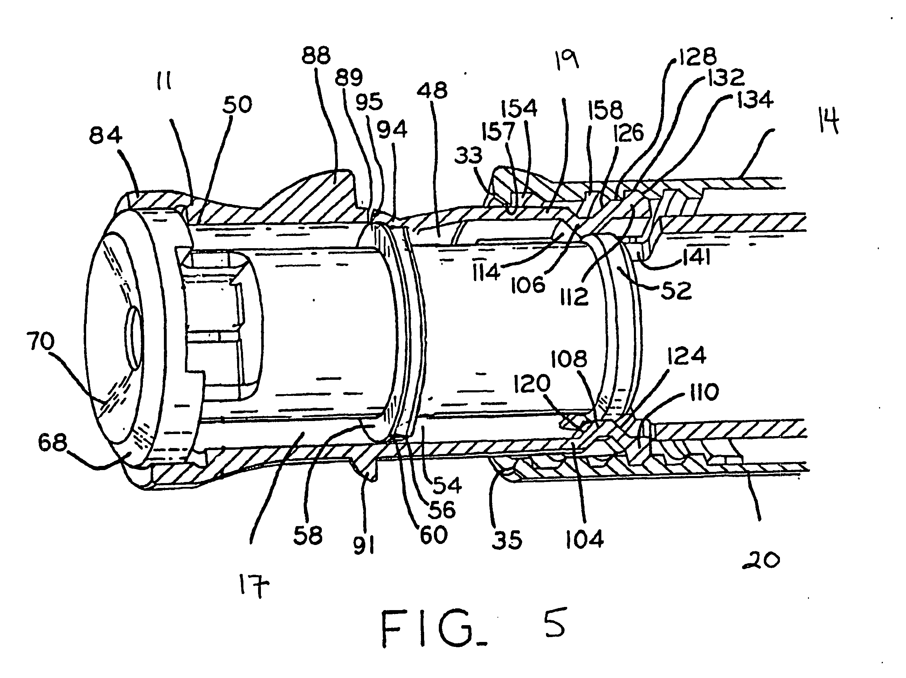

Referring to FIG. 5, disengaging device 17 comprises a hollow cylindrical portion 48 having a proximal end. Cylindrical portion 48 includes a distal end 52 in the form of an annular bead and further includes an enlarged diameter ring 54 comprising an enlarged diameter flat surface 58. The inner section of surface forms an enlarged diameter shoulder surface 60. Proximal end of disengaging device 17 further includes a finger-engageable end 68 having a recessed surface 70. End 68 is integrally connected to hollow cylindrical portion 48 by connection portions 72 (FIG. 4). Proximal end 51 includes a surface 74 (FIG. 4) that is formed from reduced length portion 76.

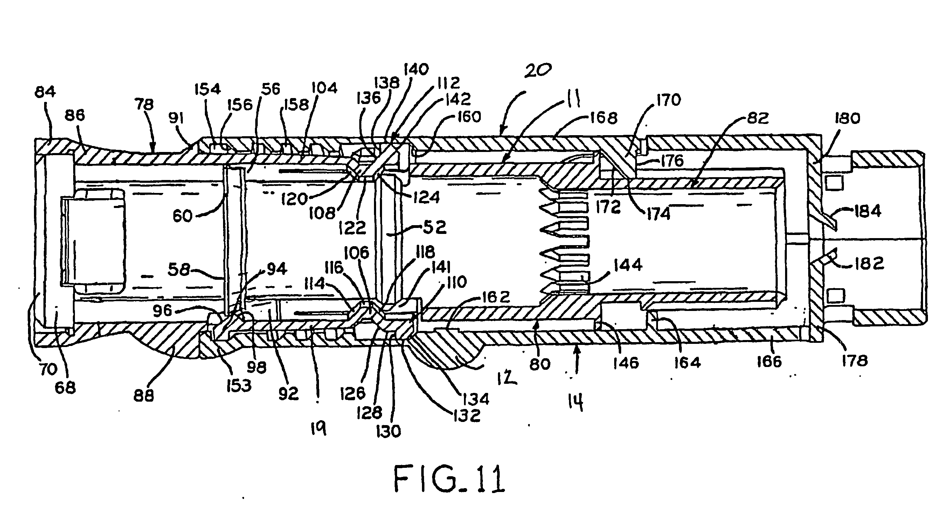

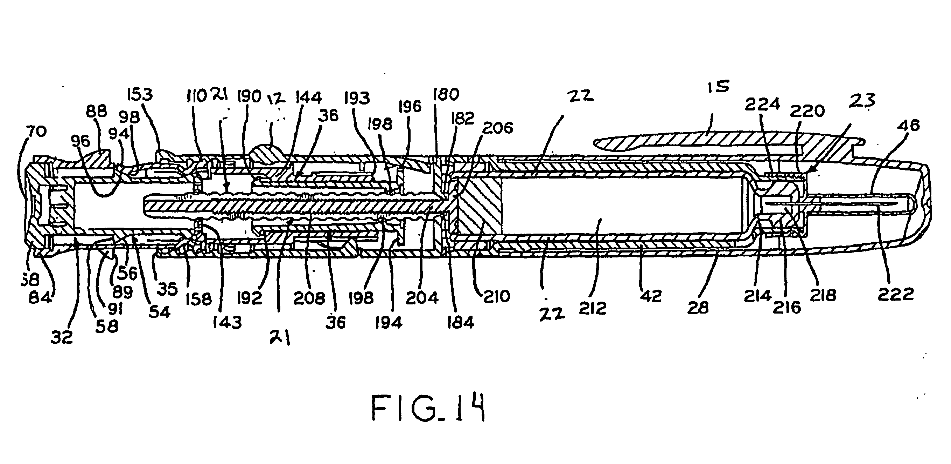

Referring to FIGS. 4 and 11, dose knob 11 is shown in detail. Dose knob 11 is generally cylindrical in shape and is hollow throughout its axial length. The diameter of dose knob 11 is at a maximum at its proximal end and is at a minimum at its distal end. Referring to FIG. 4, dose knob 11 comprises a proximal portion 78, an intermediate portion 80, and a distal portion 82. Proximal portion 78 comprises an enlarged diameter portion 84, a tapered portion 86, and an end-of-dose ring 91 extending about the circumference of proximal portion 78 as shown in FIG. 4. Ring 91 includes a bottom surface 89 (FIG. 14) that constitutes a stop surface when engaged with the rear of the housing. Ring 91 also includes an enlarged "zero-dose" protrusion 88. The proximal inner surface of flexible section 92 includes a tapered surface 96 adapted for engagement with tapered surface 56 of disengaging device 17 and a corresponding tapered surface 98. Surfaces 96 and 98 define the inner surface of finger 94.

Proximal portion 78 of dose knob 11 further includes a first U-shaped groove 100 (FIG. 4) and a second U-shaped groove (not shown) which form flexible legs 102, 104. Referring to FIG. 11, each leg 102, 104, includes an inwardly extending finger 106, 108, and an outwardly extending finger 110, 112, distal to the inwardly extending finger. Inwardly extending finger 106 includes proximal tapered surface 114, flat 116, and distal tapered surface 118. Likewise, finger 108 includes proximal tapered surface 120, flat 122, and distal tapered surface 124. Outwardly extending finger 110 comprises a proximal tapered surface 126, a flat 128, shoulder 130, enlarged diameter surface 132, and distal tapered surface 134. Outwardly extending finger 112 includes a proximal tapered surface 136, a shoulder 138, an enlarged diameter surface 140, and a distal tapered surface 143.

Referring to FIG. 4, a series of axial splines 142 are arranged circumferentially about the inner surface of dose knob 11 at the area where proximal portion 78 meets intermediate portion 80. The circumferential array of splines 143 is interrupted by legs 102 and 104. In one embodiment, there are ten splines 143 positioned about the inner circumference of dose knob 11. Referring to FIGS. 3 and 11, there is shown a plurality of splines 144 extending circumferentially about the proximal interior surface of intermediate portion 80 of dose knob 18. Unlike splines 143, splines 144 extend 360° about the inner circumference of intermediate portion 80. In one embodiment, eighteen splines 144 are positioned such that each spline is 20 circumferential degrees apart from an adjacent spline.

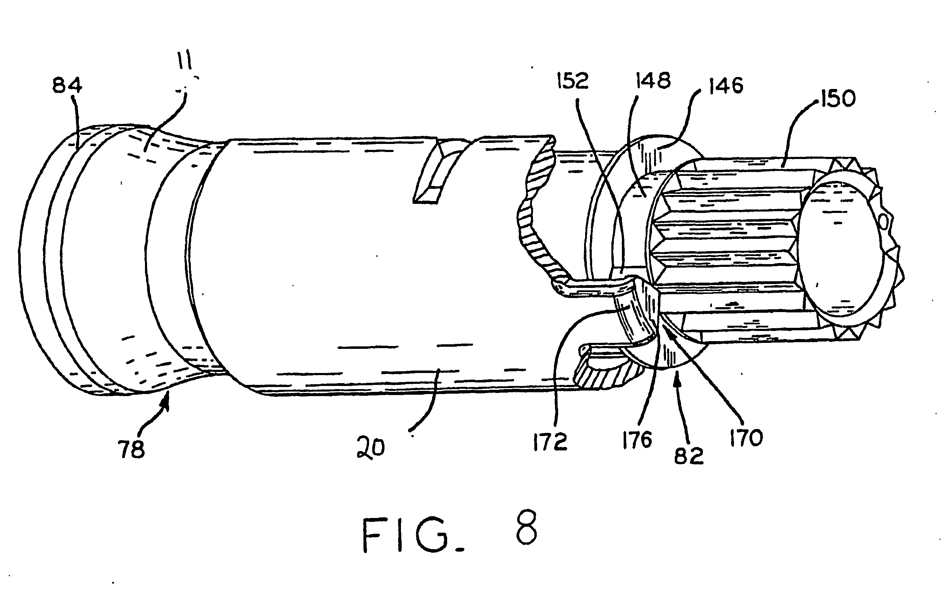

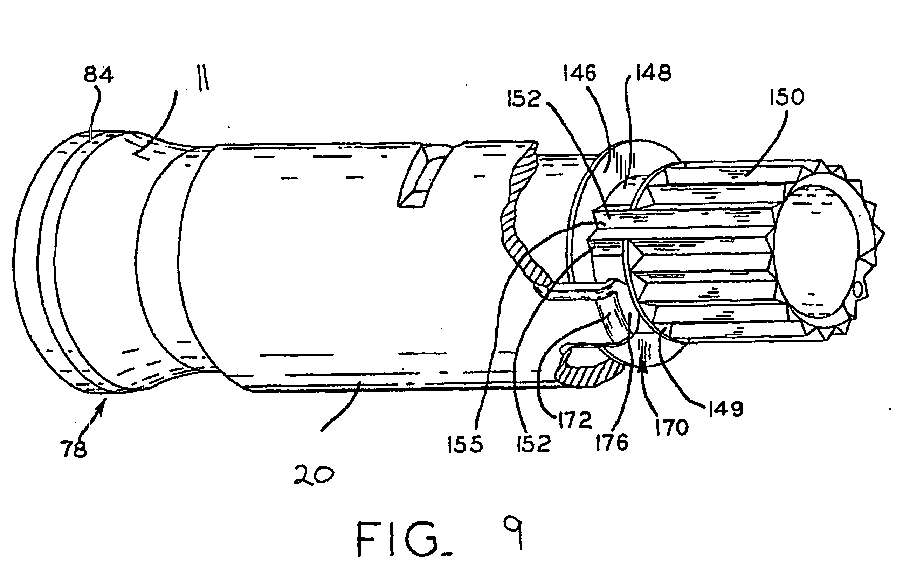

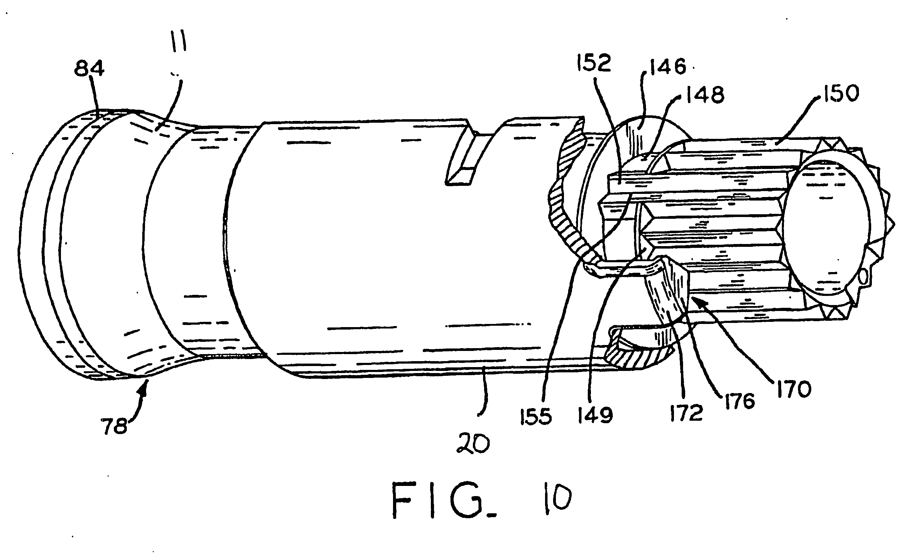

As best shown in FIGS. 8-10, distal portion 82 of dose knob 11 comprises a proximal flange 146, a reduced diameter portion 148, and a distal end comprising a series of elongated splines 150 extending externally about the circumference of distal portion 82. Splines 150 are in alignment with splines 144. Therefore, in one embodiment, there are eighteen splines 150, each corresponding to a respective spline 144. As shown in FIGS. 9 and 10, two of the splines 150 extend axially into reduced diameter portion 148. These extensions are indicated as splines 152.

Referring to FIG. 11, housing parts 14 and 26 form a proximal groove 154 having a tapered surface 156. Housing parts 14 and 20 further form a helical spiral groove 158 and a tapered circumferential surface 160 as shown in FIG. 11. Housing part 14 further includes a semicircular ridge 164 near the distal end thereof. Housing part 20 includes grooves formed therein to define a flexible leg 168 having an inwardly extending finger 170 at the end thereof. Finger 170 includes a proximal tapered surface 172, which terminates in a flat 174 and a vertical edge 176. Housing parts 14 and 20 include transverse ledges 178, 180, respectively, to reduce the diameter through the proximal end of the housing. Ledges 178 and 180 include flexible tangs 182, 184, respectively.

As best shown in FIGS. 12-14 and 18, pen device 10 further includes nut 36 and drive stem 21. Nut 36 is generally cylindrical in shape and includes a pair of axially extending grooves 186 (FIG. 2) to form resilient proximal legs 188. Each leg 188 includes a proximal raised portion 190 and two small axially extending splines 192 (FIG. 12). The distal end of nut 36 comprises an enlarged gear-like member 194 having a plurality of teeth 196 thereon. The interior surface of the distal end of nut 36 includes a helical thread 198. Thread 198 extends about 350° about the inner surface of nut 36. A groove 200 is formed at the distal end of drive stem 21 to form legs 226, 228 (FIG. 2). Ratchet teeth 204 are located on two opposing sides of drive stem 21 and axially extend along the length of drive stem 21 from groove 200 to the distal end, which constitutes plunger engagement portion 206. Helical threads 208 extend along the axial length of drive stem 21 legs 226, 228. Drive stem 21 fits within the cylindrical opening of nut 36.

As shown in FIGS. 12-15, plunger engagement portion 206 of drive stem 21 is in engagement with piston 210 of cartridge 22. Cartridge 22 is housed within cartridge retainer 42, which is permanently secured to housing parts 14 and 20. Cartridge 22 is manufactured of glass and comprises a tube defining an inner chamber 212 which openly terminates at its distal end in a neck 214 having a cap 216 including a rubber disc 218 disposed thereover. Needle assembly 23 comprises an internally threaded base 220 and a delivery needle 222. Internally threaded base 220 is threaded onto externally threaded distal portion 224 of body 42. Needle cap 46 fits over needle 222 to prevent an inadvertent insertion of needle 222 into the patient. Cap 16 snaps onto cartridge body 42 to complete the pen-like mechanism.

As described above, in order to set a dose for injection, it is first necessary to manually zero the dial from the initial radial position of the dial resulting from the previous injection. The initial axial position of dose knob 11, in a non-zero initial radial position with respect to housing part 20 is shown in FIG. 9. Specifically, finger 170 of housing part 20 is located in groove 148 of dose knob 11. Groove 148 can be rotated by rotating dose knob 11 with respect to the housing. Dose knob 11 cannot be axially retracted due to the interference between a first element on the dose metering mechanism, i.e., ledge 149 of dose knob 11, and a second element on the housing, i.e., vertical edge 176 of housing finger 170. Likewise, dose knob 11 cannot be forced axially forwardly due to the interference between surface 89 on ring 91 and end surfaces 33, 35 (FIG. 5) of housing parts 14, 20, respectively. If the user mistakenly believes that it is necessary to depress disengaging device 17 to pull out the dial, finger 94 falls into groove 154 (FIG. 11), thereby creating an interference that prevents the dial from being pulled out. Upon continued rotation of dose knob 11 with respect to housing 20, splines 152 are moved into engagement with finger 170, as shown in FIG. 8. This is the zero dose radial position of dose knob 11. This radial zero dose position is communicated to a user in four ways. The user hears a click as splines 152 engage finger 170. The movement of finger 170 over the first spline 152 into the V-shaped recess 155 between splines 152 causes a vibration in device 10 that can be felt by the user. In addition, protrusion 88 on dose knob 11 is in axial alignment with protrusion 153 of housing part 14, thereby providing a visual indication that the zero dose position has been reached. This is further visually communicated by the presence of an arrow symbol 41 in lens 12.

A series of numerals ("0", "1" and "2") are printed on the surface of intermediate portion 80 of dose knob 11. These numerals are helically spaced about the circumference of portion 80 as illustrated in FIG. 3. The lens 12 in housing part 14 is aligned with the numbers so that the appropriate number appears in the lens upon dialing up either the prime or the single dosage. A raised rectangular portion 162 (FIG. 11) of lens 12 is located at the base of lens 12 to enhance the numerals thus making them easier to read.

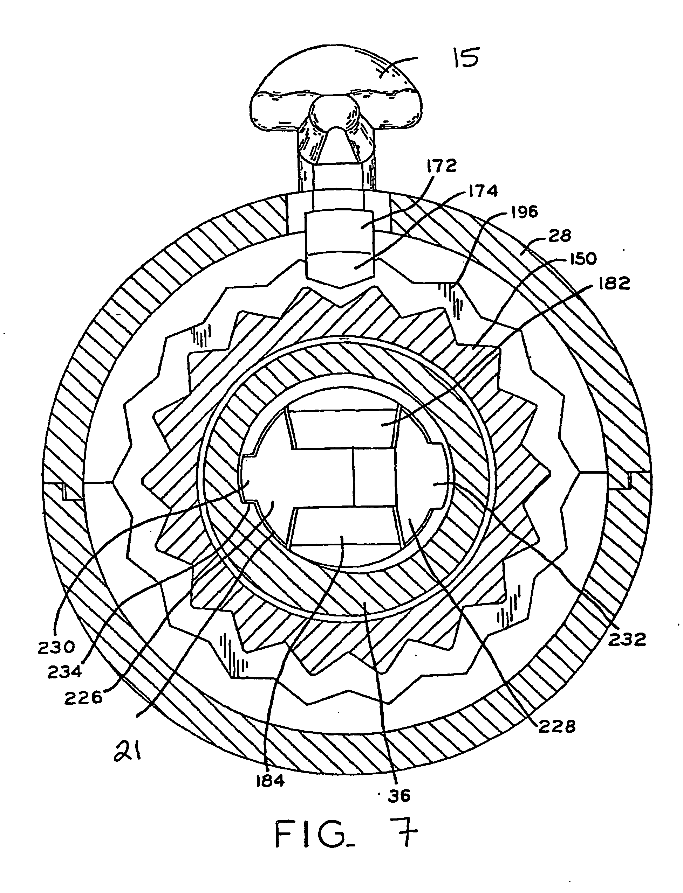

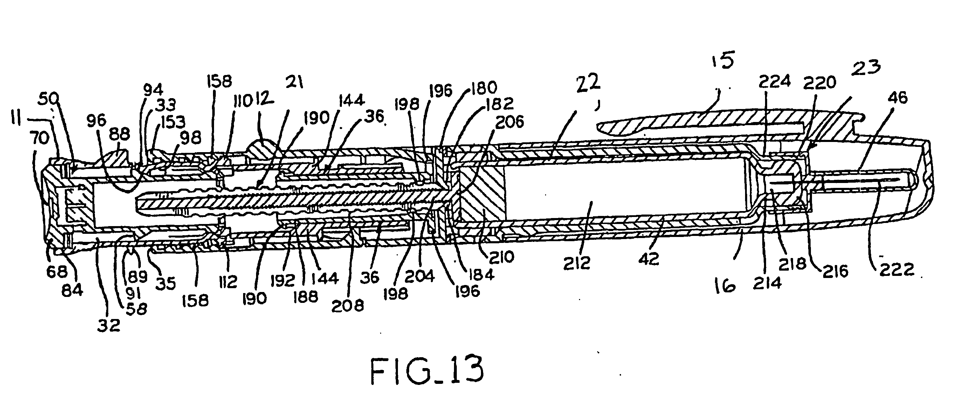

When the initial position is reached and the arrow 41 is visible, dose knob 11 may be axially retracted a predetermined distance, e.g. 3 to 5 mm, as illustrated in FIG. 13 for a dose to be set. As dose knob 11 is retracted, ledge 149 is moved past housing finger 170 resulting in housing finger 170 being in engagement with splines 150. In addition, splines 144 of dose knob 11 are moved into engagement with splines 192 of nut 36, as shown in FIG. 13. When dose knob 11 is in a dose-setting position the clutch mechanism comprised of splines 144 and 192 is engaged and rotation of dose knob 11 causes corresponding rotation of nut 36. Rotation of drive stem 21 is prevented by a key-keyway type of engagement between the anti-backup tangs 182 and 184 and drive stem 21. As shown in FIG. 7, tangs 182, 184 form a key, and drive stem 21 forms a keyway which comes into contact with the sides of the key.

Upon rotation of dose knob 11 to a positive dose radial position, fingers 110, 112 move within housing groove 158 in the proximal direction to retract dose knob 11, thereby increasing the axial distance between stop surface 89 of ring 91 and stop surfaces 33, 35 of housing parts 14, 20. Rotation of dose knob 11 causes rotation of nut 36 so that internal helical raised groove 198 of nut 36 rotates along external threads 208 of drive stem 21 to cause nut 36 to axially retract a corresponding axial distance. As shown in FIG. 10, rotation of dose knob 11 causes splines 150 to move past housing finger 170. As each spline 150 moves past finger 170, it causes a "click" to occur, thereby providing an audible indication as the priming or dose setting is reached.

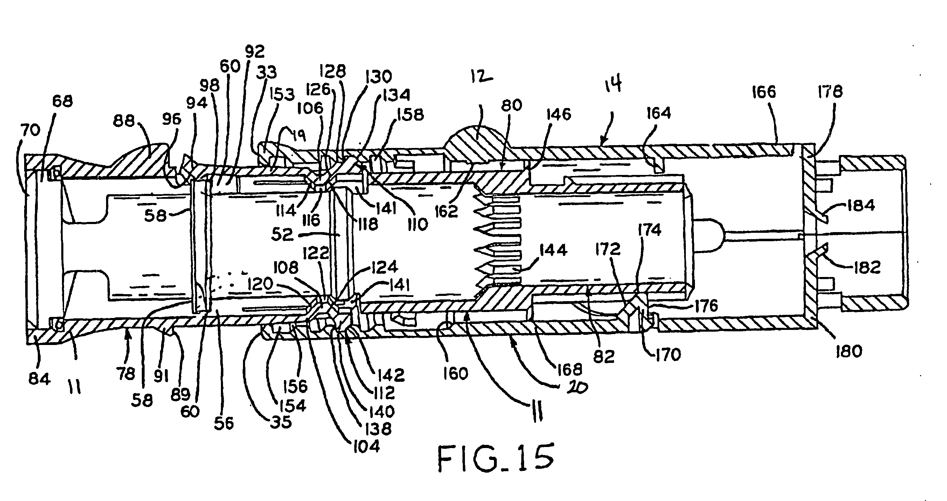

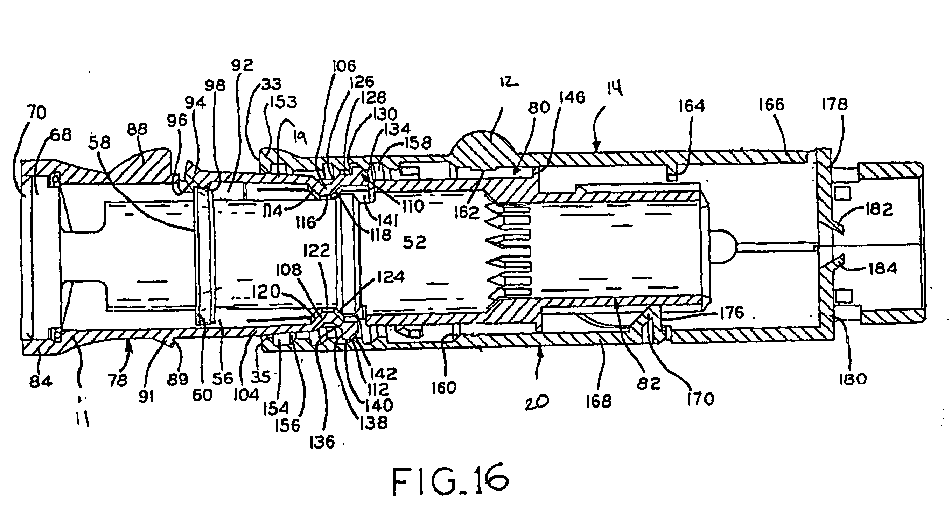

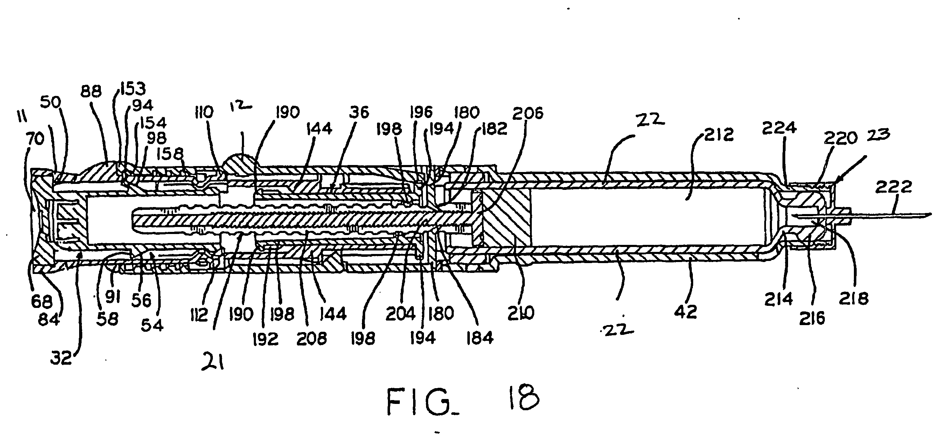

Once the priming setting has been set, cap 16 is removed and needle cover 46 is removed to expose needle 222. The needle is pointed upward, and recessed surface 70 of disengaging device 17 is pushed to perform an air shot to prime the pen, with the pushing of recessed surface 70 causing the pen to mechanically operate as described more fully below with respect to the dose injecting. Specifically, after the air shot priming, once the dose setting has been set, the needle is inserted into the patient, and recessed surface 70 of disengaging device 17 is pushed. FIGS. 15-17 illustrate the initial stages of the injection process. Referring to FIG. 15, as surface 70 is pushed, disengaging device 17 moves forwardly independently of dose knob 11 until button distal surface 52 bottoms out against internal dial shoulder 141. Thereafter, disengaging device 17 and dose knob 11 are moved together. Referring to FIG. 16, as dose knob 11 begins to move forwardly, tapered finger surfaces 134, 142 are forced out of their respective threads 158. This causes fingers 110, 112 to flex radially inwardly. As disengaging device 17 is further pressed, fingers 110, 112 move out of respective threads 158, as shown in FIG. 15. As disengaging device 17 continues to be pressed, fingers 110, 112 move into and out of the remaining threads 158 in a like manner until dial 18 reaches its end of dose position shown in FIGS. 11 and 18. The movement of edge 95 (FIG. 4) of dial finger 94 past housing edge 157 (FIG. 5) and into groove 154 (FIG. 11) creates an audible "click" sound, thereby providing an audible confirmation that the priming dose or the injectable dose has been completed. Finger 94 is in close proximity to stop surfaces 89 and 33, 35. Thus, as described above, it is the non-rotational axial advancement of the dosage metering mechanism, which drives the drive stem and thereby delivers the selected dosage.

As dose knob 11 is initially moved forwardly the clutching mechanism comprised of splines 144 and 192 decouples as splines 144 move out of engagement with splines 192 of nut 36 to rotatably disengaged dose knob 11 from nut 36 prior to any axial movement of nut 36. Dose knob 11 moves axially with respect to nut 36 until the distal end 193 (FIG. 14) of dose knob 11 engages nut flange 194 and moves nut 36 and drive stem 21 forwardly to deliver the set dosage of fluid.

Referring to FIGS. 11 and 18, forward movement of dose knob 11 and nut 36 is limited by the engagement of surface 89 of ring 91 with proximal end surfaces 33, 35 of housing parts 14, 20, respectively, as shown in FIG. 15. Referring to FIG. 15, there is a small clearance, e.g. 0.4 millimeters, between nut gear or flange 194 and internal ledges 178, 180 of housing parts 14, 20, respectively. In another embodiment, the end-of-dose stop may be designed to occur between nut flange 194 and ledges 178, 180.

Movement of drive stem 21 is prevented in the proximal direction due to anti-backup tangs 182, 184 being in engagement with ratchet teeth 204. This assures that head 206 of drive stem 21 remains at all times in constant engagement with piston 210.

Once a dosage has been completed, the user releases his finger from recessed surface 70. Upon releasing pressure from surface 70, the flexible fingers or springs 62, 64 return from their stressed conditions back to their relaxed conditions, thereby automatically retracting the disengaging device 17 back to the automatic lockout position shown in FIG. 12 to prevent the dose knob 11 from being inadvertently advanced when it is again moved to its retracted position.

As discussed, the inventive pen device and the method of its use incorporates a reservoir containing a solution of PTH. As active ingredient, the composition or solution may incorporate the full length, 84 amino acid form of parathyroid hormone, particularly the human form, hPTH (1-84), obtained either recombinantly, by peptide synthesis or by extraction from human fluid. The composition or solution may also incorporate as active ingredient fragments or variants of fragments of human PTH or of rat, porcine or bovine PTH that have PTH activity.

The parathyroid hormone fragments desirably incorporate at least the first 28 N-terminal residues, such as PTH(1-28), PTH(1-31), PTH(1-34), PTH(1-37), PTH(1-38) and PTH(1-41). Alternatives in the form of PTH variants incorporate from 1 to 5 amino acid substitutions that improve PTH stability and half-life, such as the replacement of methionine residues at positions 8 and/or 18 with leucine or other hydrophobic amino acid that improves PTH stability against oxidation and the replacement of amino acids in the 25-27 region with trypsin-insensitive amino acids such as histidine or other amino acid that improves PTH stability against protease. Other suitable forms of PTH include PTHrP, PTHrP(1-34), PTHrP(1-36) and analogs of PTH or PTHrP that activate the PTH1 receptor. These forms of PTH are embraced by the term "parathyroid hormone" as used generically herein. The hormones may be obtained by known recombinant or synthetic methods.

A preferred hormone for use in the invention is human PTH(1-34), also known as teriparatide. This particular PTH can be used as stabilized solutions of human PTH(1-34). A stabilized solution of a parathyroid hormone can include a stabilizing agent, a buffering agent, a preservative, and the like. The stabilizing agent incorporated into the solution or composition includes a polyol which includes a saccharide, preferably a monosaccharide or disaccharide, e.g., glucose, trehalose, raffinose, or sucrose; a sugar alcohol such as, for example, mannitol, sorbitol or inositol, and a polyhydric alcohol such as glycerin or propylene glycol or mixtures thereof. A preferred polyol is mannitol or propylene glycol. The concentration of polyol may range from about 1 to about 20 wt-%, preferably about 3 to 10 wt-% of the total solution.

The buffering agent employed in the solution or composition of the present invention may be any acid or salt combination which is pharmaceutically acceptable and capable of maintaining the aqueous solution at a pH range of 3 to 7, preferably 3-6. Useful buffering systems are, for example, acetate, tartrate or citrate sources. Preferred buffer systems are acetate or tartrate sources, most preferred is an acetate source. The concentration of buffer may be in the range of about 2 mM to about 500 mM, preferably about 2 mM to 100 mM.

The stabilized solution or composition of the present invention may also include a parenterally acceptable preservative. Such preservatives include, for example, cresols, benzyl alcohol, phenol, benzalkonium chloride, benzethonium chloride, chlorobutanol, phenylethyl alcohol, methyl paraben, propyl paraben, thimerosal and phenylmercuric nitrate and acetate. A preferred preservative is m-cresol or benzyl alcohol; most preferred is m-cresol. The amount of preservative employed may range from about 0.1 to about 2 wt-%, preferably about 0.3 to about 1.0 wt-% of the total solution. The stabilized teriparatide solution can contain mannitol, acetate and m-cresol with a predicted shelf-life of over 15 months at 5°C.

The parathyroid hormone is formulated for administering a dose effective for increasing toughness and/or stiffness of one or more of a subject's bones. Preferably, an effective dose provides an improvement in cortical bone structure, mass, and/or strength. An effective dose of hPTH(1-34) is typically greater than about 6 micrograms per day, preferably in the range of 15 to 65 micrograms per day, more preferably 20 to 40 micrograms per day. Doses of other PTH forms having equivalent activity in standard bioassays may also be used. A subject suffering from hypoparathyroidism can require additional or higher doses of a parathyroid hormone. Doses required for replacement therapy in hypoparathyroidism are known in the art.

The hormone can be administered regularly (e.g., once or more each day or week), intermittently (e.g., irregularly during a day or week), or cyclically (e.g., regularly for a period of days or weeks followed by a period without administration). Preferably PTH is administered once daily for a period ranging from 3 months for up to 3 years in osteoporotic patients. Typically, the benefits of administration of a parathyroid hormone persist after a period of administration. The benefits of several months of administration can persist for as much as a year or two, or more, without additional administration.

It will be appreciated by those skilled in the art that the foregoing is presented by way of illustration only, and not by way of any limitation, and that various alternatives and modifications may be made to the illustrated embodiment without departing from the scope of the invention, as defined in the claims.

## Claims

1. A pen delivery device (10) comprising;
a pen-shaped injector body (14, 20, 42) having proximal and distal ends and a reservoir (22) within the injector body;
a rotatable dose knob (11) attached to the proximal end of the injector body; and
a pen needle (23) removably attached to the distal end of the injector body;
wherein said injector body has a first hard stop (44) to prevent dose delivery of more than a preset single maximum injectable dose
**characterized in that** parathyroid hormone is contained within the reservoir; and
wherein the dose knob is marked with step designations (40) for a step-by-step method of administering the parathyroid hormone, said step designations including only two dosage step designation corresponding to two respective predetermined dosages, said two dosage step designations being a first dosage step designation corresponding to a single predetermined priming dosage, and a second dosage step designation corresponding to a single injection dosage, which single injection dosage is the preset single maximum injectable dose.

2. The pen delivery device (10) of Claim 1 where the pen delivery device is disposable.

3. The pen delivery device (10) of Claim 1 where the pen delivery device is reusable.

4. The pen delivery device (10) of any one of claims 1 to 3 wherein the reservoir (22) is a glass cartridge.

5. The pen delivery device (10) of Claim 4 where the cartridge (22) is removable from the injector body.

6. The pen delivery device (10) of Claim 4 where the cartridge (22) is permanently affixed within the injector body.

7. The pen delivery device (10) of any one of the preceding claims, wherein the dose knob (11) can be dialed in both a forward and reverse direction before dosing.

8. The pen delivery device (10) of any one of the preceding claims having a housing (14, 20) with threads (43) in communication with a dose knob (11), where said first hard stop (44) is positioned in the threads to limit rotation of the dose knob.

9. The pen delivery device (10) of Claim 8 where the step designations (40) are the single priming position indicator and the single predetermined injectable dose postion indicator on the dose knob (11).

10. The pen delivery device (10) of any one of the preceding claims wherein the injector body has a second hard stop (33, 35) to signal completion of the injection.

11. The pen delivery device (10) of any one of the preceding claims wherein the parathyroid hormone is human PTH (1-34) .

## Patentansprüche

1. Ein stiftartiges Abgabegerät (10) umfassend:
einen stiftartigen Injektorkörper (14,20,42) mit hinteren und vorderen Enden und einem Reservoir (22) in dem Injektorkörper;
einen drehbaren Dosierknopf (11), der am hinteren Ende des Injektorkörpers angebracht ist; und
eine Stiftnadel (23), die am vorderen Ende des injektorkörpers lösbar angebracht ist,
wobei der Injektorkörper einen ersten harten Anschlag (44) zur Verhinderung einer Dosisabgabe von mehr als einer vorgegebenen, maximal injizierbaren Einzeldosis aufweist,
**dadurch gekennzeichnet, dass** in dem Reservoir Parathhormon enthalten ist, und
der Dosierknopf mit Schrittbezeichnungen (40) für ein Schritt-für-Schritt-Verfahren zur Verabreichung des Parathhormons markiert ist, die nur zwei Dosierschrittbezeichnungen entsprechend zwei entsprechenden vorbestimmten Dosen enthalten, wobei die zwei Dosisschrittbezeichnungen eine erste Dosisschrittbezeichnung entsprechend einer einzelnen, vorbestimmten Anfangsdosierung und eine zweite Dosisschrittbezeichnung entsprechend einer einzelnen Injektionsdosis enthalten, welch letztere die vorgegebene, maximal injizierbare Einzeldosis ist.

2. Stiftartiges Abgabegerät (10) nach Anspruch 1, bei dem das stiftartige Abgabegerät eine Einwegvorrichtung ist.

3. Stiftartiges Abgabegerät (10) nach Anspruch 1, bei dem das stiftartige Abgabegerät wiederverwendbar ist.

4. Stiftartiges Abgabegerät (10) nach einem der Ansprüche 1 bis 3, bei dem das Reservoir (22) eine Glaspatrone ist.

5. Stiftartiges Abgabegerät (10) nach Anspruch 4, bei dem die Patrone (22) aus dem Injektorkörper entnehmbar ist.

6. Stiftartiges Abgabegerät (10) nach Anspruch 4, bei dem die Patrone (20) dauerhaft in dem Injektorkörper fixiert ist.

7. Stiftartiges Abgabegerät (10) nach einem der vorhergehenden Ansprüche, bei dem der Dosierknopf (10) vor dem Dosieren sowohl in Vorwärts- als auch in Rückwärtsrichtung gedreht werden kann.

8. Stiftartiges Abgabegerät (10) nach einem der vorhergehenden Ansprüche, enthaltend ein Gehäuse (14,20) mit Gewindegängen (43) in Verbindung mit einem Dosierknopf (11), wobei der erste harte Anschlag (44) in den Gewindegängen angeordnet ist, um die Drehung des Dosierknopfes zu begrenzen.

9. Stiftartiges Abgabegerät (10) nach Anspruch 8, bei dem die Schrittbezeichnungen (40) der Indikator für die einzelne Anfangsposition und der Indikator für die vorbestimmte, injizierbare Einzeldosis am Dosierknopf (11) sind.

10. Stiftartiges Abgabegerät (10) nach einem der vorhergehenden Ansprüche, bei dem der Injektorkörper einen zweiten harten Anschlag (33,35) zur Signalisierung des Abschlusses der Injektion aufweist.

11. Stiftartiges Abgabegerät (10) nach einem der vorhergehenden Ansprüche, bei dem das Parathhormon Human-PTH(1-34) ist.

## Revendications

1. Dispositif de délivrance de type stylo (10) comprenant :
un corps d'injecteur en forme de stylo (14, 20, 42) comportant des extrémités proximale et distale et un réservoir (22) à l'intérieur du corps d'injecteur ;
un bouton de dosage rotatif (11) attaché à l'extrémité proximale du corps d'injecteur ; et
une aiguille de stylo (23) fixée de façon détachable à l'extrémité distale du corps d'injecteur ;
dans lequel ledit corps d'injecteur comporte une première butée dure (44) pour éviter la délivrance d'une dose supérieure à une dose injectable maximale unique préréglée ;
**caractérisé en ce que** de l'hormone parathyroïde est contenue à l'intérieur du réservoir ; et
dans lequel le bouton de dosage est repéré par des désignations d'étapes (40) pour un procédé étape par étape d'administration de l'hormone parathyroïde, lesdites désignations d'étapes incluant seulement deux désignations d'étapes de dosage correspondant à deux posologies prédéterminées respectives, lesdites deux désignations d'étapes de dosage étant une première désignation d'étape de dosage correspondant à une posologie de sensibilisation prédéterminée unique, et une seconde désignation d'étape de dosage correspondant à une posologie d'injection unique, laquelle posologie d'injection unique est la dose injectable maximale unique préréglée.

2. Dispositif de délivrance de type stylo (10) selon la revendication 1, dans lequel le dispositif de délivrance de type stylo est jetable.

3. Dispositif de délivrance de type stylo (10) selon la revendication 1, dans lequel le dispositif de délivrance de type stylo est réutilisable.

4. Dispositif de délivrance de type stylo (10) selon l'une quelconque des revendications 1 à 3, dans lequel le réservoir (22) est une cartouche en verre.

5. Dispositif de délivrance de type stylo (10) selon la revendication 4, dans lequel la cartouche (22) est amovible du corps d'injecteur.

6. Dispositif de délivrance de type stylo (10) selon la revendication 4, dans lequel la cartouche (22) est fixée de façon permanente à l'intérieur du corps d'injecteur.

7. Dispositif de délivrance de type stylo (10) selon l'une quelconque des revendications précédentes, dans lequel le bouton de dosage (11) peut être tourné dans les deux sens, vers l'avant et vers l'arrière, avant dosage.

8. Dispositif de délivrance de type stylo (10) selon l'une quelconque des revendications précédentes, comportant un logement (14, 20) avec des filets (43) en communication avec un bouton de dosage (11), dans lequel ladite première butée dure (44) est positionnée dans les filets pour limiter la rotation du bouton de dosage.

9. Dispositif de délivrance de type stylo (10) selon la revendication 8, dans lequel les désignations d'étapes (40) sont l'indicateur de position de sensibilisation unique et l'indicateur de position de dose injectable prédéterminée unique sur le bouton de dosage (11).

10. Dispositif de délivrance de type stylo (10) selon l'une quelconque des revendications précédentes, dans lequel le corps d'injecteur comporte une seconde butée dure (33, 35) pour signaler l'achèvement de l'injection.

11. Dispositif de délivrance de type stylo (10) selon l'une quelconque des revendications précédentes, dans lequel l'hormone parathyroïde est la PTH (1-34) humaine.
